**Europäisches Patentamt**

**European Patent Office**

**Office européen des brevets**

(19)

(11) Publication number : **0 286 581 B1**

(12)
# EUROPEAN PATENT SPECIFICATION

(45) Date of publication of patent specification :
**05.08.92 Bulletin 92/32**

(51) Int. Cl.⁵ : **A61K 31/565,** A61K 47/00,
A61K 9/20

(21) Application number : **88730081.2**

(22) Date of filing : **08.04.88**

(54) **Buccal administration of estrogens.**

(30) Priority : **10.04.87 US 37273**

(43) Date of publication of application :
**12.10.88 Bulletin 88/41**

(45) Publication of the grant of the patent :
**05.08.92 Bulletin 92/32**

(84) Designated Contracting States :
**AT BE CH DE ES FR GB GR IT LI LU NL SE**

(56) References cited :
**EP-A- 0 107 941**
**EP-A- 0 232 877**
**WO-A-87/04342**
**FR-M- 6 955**
**US-A- 2 698 822**
**US-A- 3 009 857**

(73) Proprietor : **ZETACHRON, INC.**
**Post Office Box 339, 100 North Science Park Road**
**State College, PA 16803 (US)**

(72) Inventor : **Keith, Alec D.**
**539 Beaumont Drive**
**Boalsburg Pennsylvania 16801 (US)**
Inventor : **Snipes, Wallace C.**
**Deepwood Drive**
**Pine Grove Mills Pennsylvania 16868 (US)**

(74) Representative : **UEXKÜLL & STOLBERG**
**Patentanwälte**
**Beselerstrasse 4**
**W-2000 Hamburg 52 (DE)**

## Description

This invention relates to the use of estrogens for the preparation of a medicament for the treatment or prevention of postmenopausal syndrome and osteoporosis in the form of a buccal dosage form.

In medicinal treatment of disease or deficiency conditions wherein a pharmaceutical compound is administered to a patient by a systemic method of administration, e.g., orally, intravenously, the efficacy of the treatment is related to the concentration of drug to which the target organ is exposed, i.e., to the concentration of the drug in the circulating blood plasma. The plasma concentration or blood level of the drug is a function of a number of factors, including the rate of absorption of the drug, the rate of its metabolism an/or excretion and its tendency to be bound by substances present in the plasma or tissues. When a systemically administered drug is given in a suitable oral dosage form, which is the most common method, the dosage form passes into the gastrointestinal tract where it releases the drug into the intestinal fluid at a rate which is determined by a number of factors including the formulation and structure of the dosage form, the solubility of the drug and the pH of the intestinal fluid. The dissolved drug must then be absorbed by the lining of the intestines and transferred to the blood stream. Generally the drug is then carried by the portal system to the liver where it may be acted upon by the many enzyme systems present in that organ. Only after this process is the drug carried by the plasma in the general circulation to the rest of the body, whereby it eventually reaches the target organ. Because several steps are involved in intestinal absorption and subsequent distribution of a drug in the plasma, the oral dose of a drug which produces an effective plasma level often has to be relatively large. Furthermore, the sequence of several physiological processes involved in absorption of pharmaceutical compounds administered by the oral route results in a relatively slow rate of supply to the plasma, and consequently the time to attain peak plasma level is relatively long. This in turn requires careful attention to the dosage schedule in order to maintain an effective plasma level in the face of the processes which eliminate the drug from the bloodstream.

In the case of oral administration of estrogens, 17-beta-estradiol is rapidly metabolized in the liver. When 17-beta-estradiol is administered by the oral route the dose given is relatively large, typically 1000 or 2000 micrograms. Alternative routes of administration, such as intravenous or intramuscular injection, have been used, but these parenteral methods have obvious disadvantageous: Attempts have also been made to administer 17-beta-estradiol in a sublingual dosage form. By this route attaining effective plasma levels has required the administration of at least 200 micrograms of 17-beta-estradiol in a rapidly dissolving sublingual dosage form.

U.S. Patent 4,572,832, to Kigasawa et al., discloses that many pharmaceutically active compounds, including estrogens, can be administered via buccal dosage forms, which can be placed in various positions within the oral cavity. However, this patent does not disclose the administration of small amounts of estrogens via the buccal route to obtain therapeutic blood levels of these compounds quickly.

Pitha, U.S. Patent 4,596,795, discloses transmucosal administration of estrogens, such as 17-beta-estradiol, administered to the oral cavity in the form of a tablet containing a complex of the hormone with a cyclodextrin. Pitha's disclosure appears to be directed to administration of estrogens into the sublingual portion of the buccal cavity. This reference does not disclose administration of estrogens in a buccal dosage form comprised of a polyethylene glycol or a mixture of polyethylene glycols, and contains no specific disclosure of administration of hormones to the vestibule of the buccal cavity.

Hence a need has continued to exist for an improved medicament for rapidly and efficiently attaining plasma levels of estrogens for the treatment or prevention of postmenopausal syndrome and osteoporosis.

The subject matter of the present application refers to use of an estrogen selected from 17-β-estradiol and ethinyl estradiol and pharmaceutically active esters thereof in combination with a pharmaceutically acceptable non-toxic solid excipient for the preparation of a medicament in the form of a buccal dosage form for the administration to the vestibule of the buccal cavity for the treatment or prevention of post-menopausal syndrome and osteoporosis, wherein the dosage form comprises not more than 150 μg of the estrogen in the form of a very fine dispersion or solution in a polyethylene glycol matrix.

The cavity of the mouth (oral cavity or buccal cavity) is comprised of two parts: an outer, smaller portion, the vestibule; and an inner larger part called the mouth cavity proper. The vestibule is a collapsed slit-like space bounded internally by the gums and teeth and externally by the cheek and lips. The vestibule may be further described as having maxillary portions comprising the upper jaw and mandibular portions comprising the lower jaw. Each of these comprises a frontal space between the teeth and gums and the lips and a lateral space located between the teeth and gums and the cheek on each side. The vestibule receives secretions from the paired parotid salivary gland ducts located lateral and dorsal in the maxillary vestibule. This salivary secretion flows over the molars and into the mouth cavity proper. In addition, submandibular (submaxillary) and sublingual salivary glands discharge their secretions into the lower portion of the mouth cavity proper inside the arch formed by the lower teeth. Accordingly, the different portions of the buccal cavity experience different flows of

2

saliva depending on their anatomical relation to the salivary gland ducts.

Consequently, buccal dosage forms which are placed within the mouth cavity proper are exposed to the secretions from all three pairs of salivary glands, and medications released in that location are relatively rapidly washed out of the oral cavity. The flow of saliva in the vestibule of the oral cavity is substantially less than in the mouth cavity proper, and medications released from buccal dosage forms placed within the vestibule of the oral cavity are therefore retained in contact with the oral mucosa for longer periods of time. Dosage forms contained in the frontal mandibular vestibule receive the lowest level of fluid flow of any place in the general cavity of the mouth.

According to the invention it has been found that adequate plasma levels of 17-beta-estradiol or ethinyl estradiol for the treatment or prevention of post-menopausal syndrome and osteoporosis are attained when a dose of 17-beta-estradiol or ethinyl estradiol comprising not more than 150 micrograms of 17-beta-estradiol is used for the preparation of a medicament for administering to the vestibule of the buccal cavity. The estrogen which is brought into contact with the oral mucosa in this region of the oral cavity is absorbed readily through the mucosa and diffuses into the underlying capillary bed from which it enters the bloodstream and is distributed throughout the body. Since a drug administered via the buccal mucosa does not pass immediately through the liver as does a drug absorbed from the intestines, the metabolism which destroys a large portion of 17-beta-estradiol in its first pass through the liver is avoided by this route of administration. At the same time, the greater efficiency of absorption of 17-beta-estradiol through the buccal mucosa as compared with absorption when the drug is administered via the sublingual route permits the dosage to be reduced substantially over that required for sublingual administration. Generally, attaining effective plasma levels of 17-beta-estradiol by sublingual administration requires a dose of at least 200 micrograms. According to this invention, however, effective plasma levels of 17-beta-estradiol are attained with administration of a dose of not more than 150 micrograms, preferably 100 micrograms of 17-beta-estradiol. A dose of fifty micrograms of 17-beta-estradiol results in a plasma level therapeutic for post-menopausal syndrome and the osteoporosis. While it is uncertain why the use in a buccal dosage form according to this invention is more efficient than other transmucosal administration of 17-beta-estradiol, it is believed that the medication is in contact with the mucosa for a longer period of time when administered to the vestibule than when administered sublingually because of the lower rate of salivary flow in the vestibular site.

Within the vestibule of the buccal cavity itself, certain sites are preferred, since it has been found that the rate of flow of saliva which washes the drug away from the point of absorption varies from one location to another within the vestibule. For example the upper vestibule, between the upper gums and the cheek is less preferred because saliva flowing from this location passes to the interior portion of the oral cavity inside the teeth and thus carries dissolved medication away from the buccal mucosa. The lower vestibule between the lower gums and cheek is a preferred location because the flow of saliva which carries away the dissolved drug to the interior of the oral cavity and down the esophagus is less than in the upper vestibule. The most preferred location is the frontal mandibular vestibule, located between the lower front gums and the lower lip generally opposite the second lower lateral incisor and the lower canine teeth. This location has been found to have the lowest rate of flow of saliva in the buccal cavity and therefore, medication delivered to this site is absorbed by the buccal mucosa with greatest efficiency.

A further advantage of the use of estrogen in a buccal dosage form according to this invention is the rapid attaining of therapeutic plasma levels of estrogen. This is useful in the treatment of the vasomotor disturbances associated with menopause wherein rapid relief is sought.

In order to achieve efficient transmucosal transfer of the hormone in the use of this invention, which results in rapidly attaining therapeutic plasma levels, the 17-beta-estradiol or ethinyl estradiol is delivered in the form of a very fine dispersion or solution of the drug in a polyethylene glycol matrix. A preferred use is in a rapidly dissolving lozenge, containing 17-beta-estradiol dispersed in a polyethylene glycol excipient which rapidly dissolves in the saliva of the buccal cavity thereby releasing the 17-beta-estradiol into the vestibule of the buccal cavity close to the buccal mucosa through which it can diffuse to enter the circulation.

Suitable dosage forms include lozenges, disks, wafers and tablets. A preferred dosage form comprises a solid matrix of polyethylene glycol (PEG) having 17-beta-estradiol or a pharmaceutically acceptable ester of 17-beta-estradiol dissolved therein. A preferred dosage form comprises 17-beta-estradiol dissolved in a mixture of polyethylene glycols comprising a relatively low molecular weight PEG having a molecular weight not greater than 4000 daltons and a relatively high molecular weight PEG having a molecular weight of 6000 to 20,000 daltons. The proportions of low molecular weight PEG and high molecular weight PEG are chosen to provide a melting point of 50°-70°C. The drug composition for making the dosage forms is prepared by dissolving the drug in the molten mixture of PEG's, then solidifying the mixture by cooling to room temperature. Dosage forms may be prepared by introducing the molten drug composition into suitable molds and chilling the composition therein, or by casting a thin film of the composition on a chilled metal plate and cutting dosage forms of the proper size from the cast film. A preferred dosage form for practicing this invention is a wafer weighing 25-300 milligrams and containing 50-150 micrograms, preferably 100 micrograms of 17-beta-estradiol.

The desired plasma level of estrogen which is to be attained by the method of this invention will depend on the purpose of the administration. Estrogen is administered as replacement therapy in post-menopausal women to relieve post-menopausal syndrome and to prevent osteoporosis (see e.g. Harrison's Principles of Internal Medicine, 10th Edition, page 718). While the exact amount of estrogen needed to prevent osteoporosis is unknown, it is thought to be 50 to 100 micrograms per day, which would correspond approximately to a plasma maximum level of 250-500 picograms/milliliter of 17-beta-estradiol. For treatment of post-menopausal syndrome, it may be desirable to increase the dosage to 200-400 micrograms per day, which corresponds approximately to the daily production of estrogen in pre-menopausal women.

Evidently, the buccal administration of 17-beta-estradiol, which avoids first-pass metabolism in the liver, corresponds more nearly to the natural production of 17-beta-estradiol in the body. The rapid availability of estrogen administered by the buccal route may also be advantageous because the rapidly attained high peak values of plasma concentration tend to produce a greater saturation of the 17-beta-estradiol receptors in tissues than a lower concentration achieved, e.g., by oral administration.

The buccal route according to the invention has also been found effective for administration of esters of 17-beta-estradiol which are used in the claimed therapy, e.g., 17-beta-estradiol cypionate and 17-beta-estradiol valerate. Synthetic estrogens, such as ethinyl estradiol can also be used in this invention, since liver first pass destruction is thought to be about 70 %.

The invention will be further illustrated by the following examples.

EXAMPLE 1

The effectiveness of the buccal administration of 17-beta-estradiol is illustrated in the following example.

Buccal dosage forms were prepared by the following process. 17-Beta-estradiol was dissolved in a molten mixture of equal parts of PEG 3350 and PEG 6000 at a temperature of 60°C and the resulting solution was cast into a thin film on a chilled metal plate to form a solid thin film of a solid solution of 17-beta-estradiol in a mixed PEG matrix. Buccal dosage forms in the shape of thin wafers were cut from the film of a size to contain 100 micrograms of 17-beta-estradiol each.

A comparative test of plasma levels of 17-beta-estradiol attained by buccal versus oral administration was conducted by the following procedure. A buccal dose of 100 micrograms of 17-beta-estradiol was administered to each of four test subjects by having each subject place one of the dosage forms prepared as described above in the buccal cavity between the cheek and gum. A fifth subject received a dose of 200 micrograms in the same way. The subjects were all ovariectomized women, and the plasma estrogen levels of the subjects were monitored for a few days prior to the test and determined to be very low. 17-Beta-estradiol plasma levels were monitored periodically after the dose was administered. The results are tabulated in Table 1, and show that plasma levels of 17-beta-estradiol (E2) in these subjects increased rapidly, reaching peak concentration at 30-45 minutes after administration with peak plasma levels reaching 384 picograms/milliliter. Plasma levels decreased to near pre-treatment levels after eight hours.

After a suitable washout interval, the same four subjects were given oral doses of 500 to 1000 micrograms of 17-beta-estradiol by swallowing five to ten of the dosage forms prepared as described above. The results are also tabulated in Table 1 and show that peak plasma levels of 17-beta-estradiol were reached in 15-30 minutes after administration with average 17-beta-estradiol concentrations reaching 185 picograms./milliliter.

Table 1 also includes the data for peak values of estrone (E1), a less active metabolite of 17-beta-estradiol, and the times to attain the peak values of estrone. The data-show that for estrone also buccal administration is substantially more efficient than oral administration. The results of this experiment demonstrate that therapeutically effective plasma levels of estrogen can be achieved by buccal administration of as little as 100 micrograms of 17-beta-estradiol.

The results of this experiment indicate that the buccal route of administration is much more efficient than the oral route and can provide plasma levels of 17-beta-estradiol equivalent or greater than those achieved by oral doses five to ten times greater.

Furthermore, the results of this experiment show improvement over the administration of estrogens as complexes with cyclodextrins as taught by Pitha, U.S. Patent 4,596,795.

Figure 1 of the Pitha reference shows the results of sublingual administration of 0.5 milligrams (500 micrograms) of 17-beta-estradiol in the form of a complex with a cyclodextrin. Pitha's data show that a plasma level of about 115 picograms per milliliter was attained one hour after administration. In the experiment of this example a dose of 100-200 micrograms administered by the method of this invention produced an average plasma level of 385 picograms per milliliter 30-45 minutes after administration. Evidently, the method of administering an estrogen according to this invention is substantially more efficient since it achieves greater plasma levels with lower doses of estrogen.

## TABLE 1

| Patient/dose | Estradiol $(E_2)$ peak pg/ml | Time to estradiol $(E_2)$ peak min | Estrone $(E_1)$ peak pg/ml | Time to estrone $(E_1)$ peak min |
|---|---|---|---|---|
| W.C. | | | | |
| 200 µg buccal | 374 | 30 | 77 | 30 |
| 1000 µg oral | 265 | 15 | 136 | 120 |
| S.C. | | | | |
| 100 µg buccal | 555 | 30 | 126 | 60 |
| 800 µg oral | 375 | 15 | 349 | 60 |
| E.D. | | | | |
| 100 µg buccal | 304 | 30 | 69 | 120 |
| 500 µg oral | 123 | 15 | 81 | 60 |
| R.H. | | | | |
| 100 µg buccal | 440 | 30 | 128 | 30 |
| 800 µg oral | 94 | 30 | 200 | 120 |
| T.S. | | | | |
| 100 µg buccal | 251 | 45 | 137 | 60 |
| 800 µg oral | 69 | 30 | 173 | 240 |
| Average or range: | | | | |
| 100-200 µg buccal | 385 | 30-45 | 107 | 30-120 |
| 500-1000 µg oral | 185 | 15-30 | 188 | 60-240 |

EXAMPLE 2

This example illustrates the plasma level of estrogen attainable with a very low buccal dose.

A dose of 50 micrograms of 17-beta-estradiol was administered to a male volunteer subject by the buccal administration procedure of Example 1. The plasma level of 17- beta-estradiol reached a maximum of 290 picograms per milliliter in 30 minutes, while the estrone plasma level reached a maximum of 70 picograms per milliliter in 120 minutes. This result indicates that therapeutic levels of estrogen can be achieved using a dose of estrogen as small as 50 micrograms when administered by the method of this invention.

## Claims

1. Use of an estrogen selected from 17-β-estradiol and ethinyl estradiol and pharmaceutically active esters thereof in combination with a pharmaceutically acceptable non-toxic solid excipient for the preparation of a medicament in the form of a buccal dosage form for the administration to the vestibule of the buccal cavity for the treatment or prevention of post-menopausal syndrome and osteoporosis, wherein the dosage form comprises not more than 150 μg of the estrogen in the form of a very fine dispersion or solution in a polyethylene glycol matrix.

2. The use of claim 1 for the preparation of a medicament wherein the medicament comprises not more than 50 or 100 micrograms of said estrogen per unity of buccal dosage form.

3. The use of claims 1 or 2 for the preparation of a medicament wherein said matrix comprises a mixture of polyethylene glycols.

4. The use of claim 3 for the preparation of a medicament wherein said mixture of polyethylene glycols comprises at least one low molecular weight polyethylene glycol having a molecular weight not greater than 4000 D and at least one medium or high molecular weight polyethylene glycol having a molecular weight of 6000 to 20 000 D.

5. The use of claims 1 to 4 for the preparation of a medicament wherein the estrogen or ester thereof is in the form of a dispersion in a water-soluble solid polyethylene glycol or mixture of water-soluble solid polyethylene glycols.

6. The use of claims 1 to 4 for the preparation of a medicament wherein the dispersion is a solid solution of said estrogen or ester thereof in the water-soluble solid polyethylene glycol or mixture of water-soluble solid polyethylene glycols.

7. The use of claims 5 and 6 for the preparation of a medicament wherein said mixture of water-soluble solid polyethylene glycols is a mixture of a low molecular weight polyethylene glycols having a molecular weight not greater than 4000 D and at least one medium or high molecular weight polyethylene glycol having a molecular weight of 6000 to 20 000 D.

## Patentansprüche

1. Verwendung eines aus 17-beta-Östradiol und Ethinylöstradiol sowie pharmazeutisch wirksamen Estern davon ausgewählten Östrogens in Kombination mit einem pharmazeutisch verträglichen nicht-toxischen festen Arzneimittelträger zur Herstellung eines Medikaments in der Form einer Dosierungsform für die Mundhöhle zur Verabreichung in das Vestibulum der Mundhöhle zur Behandlung oder Prävention des post-menopausalen Syndroms und der Osteoporose, wobei die Dosierungsform nicht mehr als 150 μg des Östrogens in der Form einer sehr feinteiligen Dispersion oder Lösung in einer Polyethylenglykolmatrix umfaßt.

2. Verwendung nach Anspruch 1 zur Herstellung eines Medikaments, bei der das Medikament nicht mehr als 50 oder 100 μg des Östrogens pro Einheit der bukkalen Dosierungsform umfaßt.

3. Verwendung nach den Ansprüchen 1 oder 2 zur Herstellung eines Medikaments, bei der die Matrix eine Mischung von Polyethylenglykolen umfaßt.

4. Verwendung nach Anspruch 3 zur Herstellung eines Medikaments, bei der die Mischung von Polyethylenglykolen mindestens einen niedermolekularen Polyethylenglykol mit einem Molekulargewicht von nicht mehr als 4000 D und mindestens einen mittel- oder hochmolekularen Polyethylenglykol mit einem Molekulargewicht von 6000 bis 20 000 D umfaßt.

5. Verwendung nach den Ansprüchen 1 bis 4 zur Herstellung eines Medikaments, bei der das Östrogen oder der Ester davon als eine Dispersion in einem wasserlöslichen festen Polyethylenglykol oder einer Mischung aus wasserlöslichen festen Polyethylenglykolen vorliegt.

6. Verwendung nach den Ansprüchen 1 bis 4 zur Herstellung eines Medikaments, bei der die Dispersion eine feste Lösung des Östrogens oder Esters davon in dem wasserlöslichen festen Polyethylenglykols oder der Mischung aus wasserlöslichen festen Polyethylenglykolen ist.

7. Verwendung nach den Ansprüchen 5 und 6 zur Herstellung eines Medikaments, bei der die Mischung der wasserlöslichen festen Polyethylenglykole eine Mischung ist aus einem niedermolekularen Polyethylenglykol mit einem Molekulargewicht von nicht mehr als 4000 D und mindestens einem mittel- oder hochmolekularen Ethylenglykol mit einem Molekulargewicht von 6000 bis 20 000 D.


**Revendications**

1. Utilisation d'un oestrogène choisi parmi 17-bêta-estradiol et éthinyl estradiol et leurs esters pharmaceutiquement actifs en combinaison avec un excipient solide non toxique pharmaceutiquement acceptable, pour la préparation d'un médicament sous la forme d'une dose buccale pour l'administration à l'avant-bouche de la cavité buccale pour le traitement ou la prévention du syndrome post-ménopause et de l'ostéoporose, où la dose ne comprend pas plus de 150 μg de l'oestrogène, sous la forme d'une solution ou d'une dispersion très fine dans une matrice de polyéthylène glycol.

2. Utilisation de la revendications 1, pour la préparation d'un médicament où le médicament ne contient pas plus de 50 ou 100 microgrammes dudit oestrogène par unité de la forme de dose buccale.

3. Utilisation des revendications 1 ou 2, pour la préparation d'un médicament où ladite matrice comprend un mélange de polyéthylène glycols.

4. Utilisation de la revendication 3, pour la préparation d'un médicament où ledit mélange de polyéthylène glycols comprend au moins un polyéthylène glycol de faible poids moléculaire, ayant un poids moléculaire ne dépassant pas 4000 D, et au moins un polyéthylène glycol de poids moléculaire moyen ou élevé, ayant un poids moléculaire de 6000 à 20 000 D.

5. Utilisation des revendications 1 à 4, pour la préparation d'un médicament où l'oestrogène ou son ester est sous la forme d'une dispersion dans un polyéthylène glycol solide soluble dans l'eau ou un mélange de polyéthylène glycols solides solubles dans l'eau.

6. Utilisation des revendications 1 à 4, pour la préparation d'un médicament où la dispersion est une solide dudit oestrogène ou son ester dans le polyéthylène glycol solide soluble dans l'eau ou le mélange de polyéthylène glycols solides solubles dans l'eau.

7. Utilisation des revendications 5 et 6, pour la préparation d'un médicament où ledit mélange des polyéthylène glycols solides solubles dans l'eau est un mélange de polyéthylène glycols de faible poids moléculaire ayant un poids moléculaire ne dépassant pas 4000 D et d'au moins un polyéthylène glycol de poids moléculaire moyen ou élevé ayant un poids moléculaire de 6000 à 20 000 D.